(19) Europäisches Patentamt / European Patent Office / Office européen des brevets

(11) **EP 2 026 741 B1**

(12) **EUROPÄISCHE PATENTSCHRIFT**

(45) Veröffentlichungstag und Bekanntmachung des Hinweises auf die Patenterteilung:
**12.08.2015 Patentblatt 2015/33**

(21) Anmeldenummer: **07766722.8**

(22) Anmeldetag: **12.06.2007**

(51) Int Cl.:
*A61J 3/10* (2006.01)    *G03F 7/00* (2006.01)
*A61K 9/20* (2006.01)    *B30B 15/06* (2006.01)

(86) Internationale Anmeldenummer:
**PCT/IB2007/052216**

(87) Internationale Veröffentlichungsnummer:
**WO 2007/144826 (21.12.2007 Gazette 2007/51)**

(54) **Pharmazeutische Tabletten mit diffraktiver Mikrostruktur und Presswerkzeuge zur Herstellung solcher Tabletten**

Pharmaceutical tablets with diffractive microstructure and compression tools for producing such tablets

Comprimés pharmaceutiques ayant une microstructure à diffraction et outils de pressage pour fabriquer de tels comprimés

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **13.06.2006 US 812967 P**
**13.06.2006 US 812957 P**
**12.01.2007 CH 42072007**
**12.01.2007 CH 43072007**

(43) Veröffentlichungstag der Anmeldung:
**25.02.2009 Patentblatt 2009/09**

(73) Patentinhaber: **CSEM Centre Suisse d'Electronique et de Microtechnique SA - Recherche et Développement**
**2002 Neuchâtel (CH)**

(72) Erfinder:
• **STUCK, Alexander**
**5430 Wettingen (CH)**
• **WALTER, Harald**
**8810 Horgen (CH)**
• **SCHNIEPER, Marc**
**1213 Onex-genève (CH)**
• **SÖCHTIG, Jürgen**
**8907 Wettswil (CH)**
• **ZSCHOKKE, Christian**
**8820 Wädenswil (CH)**

(74) Vertreter: **GLN SA**
**Avenue Edouard-Dubois 20**
**2000 Neuchâtel (CH)**

(56) Entgegenhaltungen:
**EP-A- 1 357 433        WO-A-01/10464**
**WO-A-2006/047695    US-A1- 2004 163 441**
**US-A1- 2005 232 130**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

**Technisches Gebiet**

Die Erfindung betrifft eine Tablette mit einem optischen Sicherheitsmerkmal in Form einer diffraktiven Mikrostruktur, ein Presswerkzeug zur Herstellung solcher Tabletten, und ein Verfahren zum Herstellen solcher Werkzeuge.

**Stand der Technik**

Fälschungen, Graumarkt und illegale Reimporte sind ein großes Problem für Arzneimittel. Immer mehr Arzneimittel und Medikamente werden gefälscht, wobei dies nicht nur ein Problem in Entwicklungsländern ist, wo der Anteil der gefälschten Produkte in der Lieferkette manchmal bereits bei über 50% liegt.

Das Problem besteht auch in den Industrieländern, in denen die Preise von Arzneimitteln oft viel höher sind. So werden beispielsweise aus sozialen Überlegungen die Preise für AIDS- oder Krebsmedikamente in Entwicklungsländern oft deutlich gesenkt, was jedoch die Gefahr missbräuchlicher Reimporte in Industriestaaten vergrößert.

Zur Verhinderung von Missbrauch werden Verpackungen von Arzneimitteln mit fälschungssicheren Merkmalen versehen. Hologramme, optisch variable Tinten, Fluoreszenzfarbstoffe, spezielle Drucktechniken wie Mikrodruck und andere Sicherheitsmerkmale werden mit Klebeetiketten auf der Verpackung befestigt, auf den Karton laminiert oder direkt auf der Verpackung angebracht. Der Hauptnachteil solcher Kennzeichnungen besteht darin, dass sie vom Produkt oder der Verpackung entfernt und danach wieder verwendet oder analysiert werden können. Einige Unternehmen bringen Sicherheitsmerkmale auf die Versiegelungsfolie von Blisterverpackungen an, doch weisen diese die gleichen Nachteile auf.

Methoden, bei denen fälschungssichere Signaturen, wie z.B. DNA von bekannter Sequenz (US 5,451,505), Moleküle mit charakteristischen Isotopenzusammensetzung, oder Mikropartikel mit charakteristischer Farblagenfolge (US 6,455,157) beigefügt werden, sind äußerst kritisch, da diese Signaturen mit dem Arzneimittel zusammen eingenommen werden. Aus diesem Grund haben Zulassungs-Behörden, wie z.B. die Food and Drug Administration (FDA) in den USA, für solche Methoden bisher keine Bewilligung erteilt.

Einige Versuche, ein Hologramm auf essbare Produkte anzubringen, sind veröffentlicht. WO 01/10464 A1 offenbart die Beschichtung von essbaren Produkten mit einer thermisch form- und prägbaren Schicht. Da die Aufbringung dieser Schicht jedoch die Zusammensetzung sowie den Herstellungsprozess von pharmazeutischen Pillen verändert, braucht es eine neue behördliche Zulassung. Darüber hinaus ist die Erwärmung während der thermisch formenden Schritte für viele aktive Wirkstoffe problematisch.

US 4,668,523 zeigt einen anderen Ansatz, bei dem eine Polymerlösung in Kontakt gebracht wird mit einer Form mit diffraktivem Relief. Anschliessend wird das Polymer beim Trocknen gehärtet. Dieser Schritt kann durch Erhitzen beschleunigt werden. Am Ende trägt das gehärtete essbare Polymerprodukt das diffraktive Relief. Diese Methode beschränkt sich auf Polymerlösungen und ist sehr langsam. Darüber hinaus ist auch hier das Erhitzen der aktiven Wirkstoffe, die für die Herstellung pharmazeutischer Tabletten verwendet werden, problematisch. Diese Nachteile haben die Markteinführung dieser Techniken verhindert.

**Darstellung der Erfindung**

Aufgabe der vorliegenden Erfindung ist es, eine Tablette entsprechend Anspruch 1 mit integriertem Sicherheitsmerkmal zu Verfügung zu stellen, welche im wesentlichen die gleiche Zusammensetzung wie eine herkömmliche Tablette aufweist, welche ohne erhöhte Temperaturen während des Herstellungsprozesses produziert werden kann, und welche ohne Verlängerung des Produktionsprozesses gegenüber den herkömmlichen Verfahren auskommt. Eine weitere Aufgabe der Erfindung ist, ein Presswerkzeug zur Verfügung zu stellen, mit welchem solche Tabletten hergestellt werden können, sowie Verfahren zum Herstellen solcher Werkzeuge.

WO2006/047695 offenbart eine Dosierungsform, die im wesentlichen aus mindestens einem Wirkstoff besteht und einen ersten Abschnitt umfasst, der eine äußere Oberfläche und einen oder mehrere Hohlräume aufweist, die mindestens eine innere Oberfläche definieren. Des Weiteren weist der erste Abschnitt gegebenenfalls Kerben auf. Die Dosierungsform umfasst weiter einen zweiten Formteil, der in den Hohlräumen des ersten Abschnitts eingelegt ist und eine äußere Oberfläche aufweist. Der zweite Formteil trägt eine Mikrorelief.

Unter dem Begriff Tablette ist in diesem Zusammenhang nicht nur Tabletten und Pillen gemeint, die zum Schlucken, Lutschen, Kauen oder Zergehenlassen im Mund vorgesehen sind, sondern auch andere medikamentöse Darreichungsformen wie Zäpfchen/Suppositorien oder Produkte, welche vor der Einnahme in Flüssigkeiten aufgelöst werden. Ebenfalls mitgemeint sind neben pharmazeutischen Tabletten auch nicht-pharmazeutische Produkte wie beispielsweise Bonbons oder Süßstofftabletten.

Diese und andere Aufgaben werden gelöst durch eine Tablett, ein Presswerkzeug, und durch Verfahren zur Herstellung solcher Presswerkzeuge, gemäß den unabhängigen Patentansprüchen. Bevorzugte Varianten sind in den abhängigen Ansprüchen gegeben.

Eine erfindungsgemäße Tablette weist auf Ihrer Oberfläche eine diffraktive Mikrostruktur auf, welches im optischen Spektralbereich wahrnehmbare Beugungs-Effekte erzeugt, und so als Sicherheitsmerkmal dient. Die mikrostrukturierte Fläche kann auch makroskopisch strukturiert sein, um beispielsweise Logos, Markennamen etc. zu formen. Das Sicherheitsmerkmal kann nicht von der Tablette entfernt werden, und kann auch nicht nachträglich auf gefälschte Produkte übertragen werden. Zur Herstellung solcher Tabletten kann ein erfindungsgemäßes Presswerkzeug verwendet werden, bestehend aus einer Pressform und zwei Pressstempeln. Die dem zur verpressenden Pulvergemisch zugewandte Oberfläche der Pressform und/oder eines oder beider Pressstempel ist mit einer diffraktiven Mikrostruktur versehen, welche während des Pressvorgangs, genauer gesagt während dem Kompressionsund Verdichtungsprozess, auf die Oberfläche der Pulverpartikel abgebildet wird, so dass auf der Oberfläche der fertigen Tablette eine dauerhafte, diffraktive Mikrostruktur entsteht.

Zur Herstellung der erfindungsgemäßen Tabletten können die herkömmlichen Temperaturen, Druckstärken und Prozessgeschwindigkeiten von bekannten Tablettenpressen beibehalten werden. Insbesondere genügt eine Kompressionszeit pro Tablette von weit unter 100 ms. Die erfindungsgemäßen Werkzeuge können in herkömmlichen Tablettierungsmaschinen eingesetzt werden. Die Herstellung der erfindungsgemäßen Tabletten ist somit kompatibel mit den bestehenden und qualifizierten Tablettenherstellungsverfahren, und ist somit kostengünstig.

## Wege zur Ausführung der Erfindung

Im Folgenden wird die Erfindung unter Mithilfe von Zeichnungen näher erklärt.

Figur 1 zeigt eine vereinfachte schematische Darstellung des Tablettenpressprozesses.

Figur 2 zeigt einen schematischen Querschnitt von diffraktiven Mikrostrukturen auf der Oberfläche von Tabletten. Hergestellt mit dem erfindungsgemäßen Verfahren, mit (a) rechteckig, (b) sinusförmig und (c) dreieckig geformten Gitterlinien;

Figur 3 zeigt eine Aufnahme einer gepressten Tablette mit einer diffraktiven Mikrostruktur, hergestellt nach dem erfindungsgemäßen Verfahren.

Figur 3a zeigt eine schematische Darstellung von erfindungsgemäßen Tabletten mit Mikrostrukturen in Vertiefungen.

Figur 3b zeigt die schematische Darstellung eines Lesegeräts zur Authentifizierung von erfindungsgemäßen Tabletten.

Figur 4 zeigt (a) eine Aufnahme eines mikrostrukturierten Presswerkzeugs zur Verwendung im erfindungsgemäßen Verfahren und (b) ein SEM-Bild (SEM=Scanning Electron Microscope) einer nach dem erfindungsgemäßen Verfahren hergestellten Tablette.

Figur 5 zeigt schematisch die Schritte des Ionenätzverfahrens zur Herstellung eines erfindungsgemäßen Presswerkzeugs: (a) Holographiebelichtung, (b) Chromschiefbedampfung, (c) Trockenätzen, (d) fertig mikrostrukturierte Presswerkzeugoberfläche.

Figur 6 zeigt ein Beispiel einer Spannungs-/Dehnungskurve.

Figur 7 zeigt eine Aufnahme einer mittels Kaltprägung mikrostrukturierten Aluminiumplatte.

Figur 8 zeigt die schematische Darstellung eines Hämmerprozesses zur Abbildung einer diffraktiven Mikrostruktur auf einer Presswerkzeugoberfläche.

### *Pulvergemisch für pharmazeutische Tabletten*

Die meisten Tabletten werden durch Verpressen einer Pulvermischung in einer Pressform hergestellt. Werden aktive Pulver und Füllstoffe lediglich gemischt und anschließend direkt zu Tabletten gepresst, spricht man von direkter Tablettierung. Dieser Prozess ist hauptsächlich ein Hochdruckformprozess.

Die zu verpressende Mischung besteht aus Partikeln verschiedener Größe, wobei die Größenverteilung der Partikel für den Tablettenpressprozess kritisch ist. Tabelle 1 zeigt ein Beispiel einer typischen Mischung inkl. Hilfsstoffen zur Herstellung einer pharmazeutischen Tablette. Tabelle 2 zeigt die dazugehörige typische Partikel-Größenverteilung.

**Tabelle 1**

| Anteil Gew.% | Substanz |
|---|---|
| 72.75 | Laktose-Monohydrat |
| 24.25 | Mikrokristalline Cellulose |
| 1.00 | Aerosil (Kolloid-Kieselerde, getrocknet) |

(fortgesetzt)

| Anteil Gew.% | Substanz |
|---|---|
| 1.00 | Magnesium-Stearat |
| 1.00 | Natrium-Salicylat. (exemplarischer aktiver Wirkstoff) |

**Tabelle 2**

| Durchmesser der Partikel in μm | Anteil in Gew.% |
|---|---|
| < 75 | 15 - 25 |
| 75 - 150 | 30 - 50 |
| 150 - 250 | 15 - 25 |
| 250 - 500 | 5 - 15 |
| > 500 | < 2 |

Laktose und Cellulose sind die am weitesten verbreiteten Binder und Füllstoffe bei direkten Tablettierungsprozessen. Diese Substanzen sind besonders dazu geeignet, mit einer diffraktiven Mikrostruktur versehen zu werden.

Der Pulvertransport in den Tablettenpressapparaturen erfolgt durch die Schwerkraft. Somit ist ein gutes Rieselverhalten zwingend. Aerosil verbessert den Pulverfluss.

Magnesium-Stearat wird als Gleitmittel eingesetzt. Gleitmittel funktionieren, indem sie sich über die Oberfläche des Pulvers verteilen. Sie verringern die Reibungskräfte zwischen dem Pulver und den Presswerkzeugen, und verhindern so, dass die Tablette am Presswerkzeug haften bleibt.

Der Pulvermischung können Dekompositionswirkstoffe beigefügt werden, um die Dekomposition, also die Auslösung in Wasser, zu verbessern. Die Dekompositionszeit von Pillen wird typischerweise in Wasser bei 37°C gemessen. Manchmal wird ein Farbstoff hinzugefügt, jedoch sind nur wenige Farbstoffe zur Verwendung in Medikamenten zugelassen. Praktisch alle pharmazeutischen Tabletten sind deshalb matt weiß. Einige sind knallrot oder hellblau. Somit haben alle im direkten Tablettierungsprozess hergestellten Tabletten eine leuchtende und/oder lictitstreuende Oberfläche.

Für den Pressprozess sind Partikel kritisch, die größer als 500 μm bzw. kleiner als 75 μm sind. Erstere vermindern die mechanische Stabilität der gepressten Tablette, und letztere sind problematisch für den Partikelfluss während des Auffüllens des Hohlraumes des Presswerkzeugs. Somit muss der Anteil dieser Partikel möglichst klein gehalten werden. Insgesamt kann festgestellt werden, dass praktisch alle im Tablettenpressprozess verwendeten Pulverpartikel deutlich größer sind als die in die Oberfläche einzubringenden diffraktiven Mikrostrukturen, welche typischerweise Strukturen kleiner als 5 μm aufweisen.

Um eine unerwünschte chemische Veränderung der Inhaltsstoffe während der Herstellung der Tabletten zu verhindern, sollte die Temperatur vorteilhaft 50 °C, und noch besser 40 °C, nicht überschreiten. Bevorzugt beträgt die Temperatur zwischen 15 °C und 35 °C, bzw. Raumtemperatur.

*Parameter der diffraktiven Mikrostrukturen*

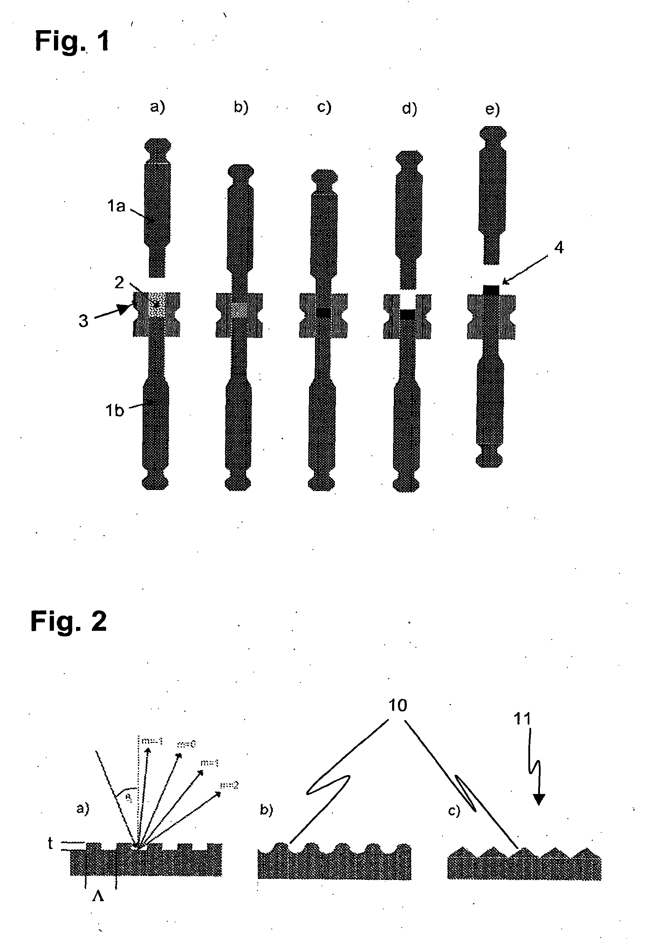

Das zuverlässige und dauerhafte Einbringen und der Erhalt typischer diffraktiver Mikrostrukturen mit einer Periode A von ca. 1-2 μm und einer Tiefe t in der Größenordnung von 200-300 nm, wie sie beispielsweise in Figur 2 dargestellt sind in die Oberfläche einer Tablette während des direkten Tablettierungsprozesses ist schwierig. Die Pulvermischungen sind natürlich nicht dafür gedacht, mikrostrukturiert zu werden, und die Größe der Mikrostrukturen ist viel geringer als die Dimension der Partikel. Aus diesem Grund muss die Oberfläche der Partikel selbst mikrostrukturiert werden. Und schließlich ist der Tablettierungsprozess nicht zuletzt so schnell, dass die Zeit für die Mikrostrukturierung extrem kurz ist. Um dies erreichen zu können, müssen gewisse Parameter der diffraktiven Mikrostruktur optimiert werden, insbesondere die als Prägemuster wirkende diffraktive Mikrostruktur auf der Werkzeugoberfläche. Die ermittelten besonders gut geeigneten Parameterbereiche der Mikrostrukturen für erfindungsgemäße Tabletten sind in Tabelle 2a zusammengefasst.

**Tabelle 2a**

| Parameter | geeigneter Bereich | bevorzugter Bereich | besonders bevorzugter Bereich |
|---|---|---|---|
| Periode $\Lambda$ | 300 - 5000 nm | 800 - 2500 nm | 1200 - 2500 nm |
| Tiefe t | 80 - 1000 nm | 100 - 500 nm | 150 - 300 nm |
| Form | rechteckige, sinusförmige, dreieckige oder gerundete Formen | sinusförmige oder gerundete Formen | sinusförmige oder gerundete Formen |

Eine Herausforderung beim Tablettierungsprozess besteht darin, zu vermeiden, dass die aus der Oberfläche der erfindungsgemässen Tablette herausragenden diffraktiven Mikrostrukturen abgebrochen werden. Mikrostrukturen, welche aus linearen Gitterlinien bestehen (1d-Gitter) sind geeigneter als Punktgitter (2d-Gitter), da die Linien eine mechanische größere Stabilität aufweisen als die Punkte. Gekreuzte Gitter mit der Form eines Lochrasters sind dank der Stabilität der verbundenen Gitterlinien ähnlich geeignet.

Die Mikrostrukturierung vergrößert die Oberfläche des Presswerkzeugs und somit die Kontaktfläche zwischen dem Presswerkzeug und der gepressten Tablette. Dies führt zu einer verstärkten Adhäsion und kann somit die Ablösung der fertigen Tablette vom Werkzeug stören. Um diesen Effekt zu minimieren, weist die Mikrostruktur vorteilhaft eine gerundete oder eine dreieckige Form auf, z.B. ein sinusförmiges Gitter (Fig. 2(b), (c)). Weniger ideal sind Mikrostrukturen mit senkrechten Wänden, wie in Fig. 2(a). Darüber hinaus sollte die Tiefe t der Mikrostrukturen so niedrig wie möglich sein. Jedoch braucht es für einen sichtbaren diffraktiven Effekt eine Mindesttiefe t von ca. 80 nm. Die Diffraktionseffizienz eines sinusförmigen Gitters ist beispielsweise maximal, wenn die Gittertiefe 0,3 - 0,4 Gitterperioden entspricht. Darüber hinaus muss die Mikrostruktur tiefer sein als die Gleitmittelschicht zwischen der Oberfläche des Presswerkzeugs oder der Pressformwand und der Tablettenmasse. Die meisten Gleitmittel haben eine laminare Struktur mit Gleitebenen, die sich leicht parallel zur Oberfläche des Presswerkzeugs oder der Pressform bewegen. Aus diesem Grund werden Mikrostrukturen, die nur in diese Gleitlage eingebracht werden, leicht abgerissen.

*Herstellung von Tabletten mit diffraktiven Mikrostrukturen*

Figur 1 zeigt schematisch den Herstellungsprozess einer Tablette. Das zu verpressende Pulver 2, ein Gemisch der pulverförmigen Bestandteile, wird in eine Pressform 3 eingebracht. Zwei axial gefluchtet angeordnete Pressstempels 1a, 1 b üben axial mechanische Kräfte aus, wodurch die Tablette entsteht.

Die auf die Tablette einzubringende diffraktive Mikrostruktur befindet sich auf der Oberfläche des Pressstempels 1 a, 1 b und/oder auf der Innenwand der Pressform 3. Ist die Wand der Pressform 3 mit einem linearen diffraktiven Gitter als Mikrostruktur versehen, sind die Gitterlinien vorzugsweise parallel zur axialen Bewegungsrichtung der Pressstempel-Werkzeuge angeordnet, um den Auswurf der fertigen Tablette 4 zu unterstützen. Dennoch ist es hinsichtlich der im Pressprozess auftretenden mechanischen Spannungen einfacher, die Mikrostruktur auf den Pressstempeln 1 a, 1 b anzubringen.

Das Pulver füllt den Hohlraum in der Pressform 3, welcher vom unteren Pressstempel 1 b verschlossen wird, siehe Fig. 1 (a). Das Volumen der Pressform definiert die Pulvermenge, die zur Tablette gepresst wird. Dieses Volumen kann durch die Position des unteren Pressstempels 1 b während des Füllens des Hohlraums eingestellt werden. Die Kompressionskraft liegt typischerweise zwischen 5-25 kN. Moderne Rotationspressen erreichen maximale Kompressionskräfte von bis zu 160 kN. Während des Pressvorgangs finden zwei zusammenhängende Phänomene gleichzeitig statt: Kompression und Konsolidation (K. Marshall, "Tablet press fundaments", Tablets & Capsules 2005, S.6-11). Ersteres führt zu einer Reduzierung des Massenvolumens, letzteres bewirkt eine Erhöhung der mechanischen Stärke der Masse. Wird nun also Kraft auf das Pulver ausgeübt, verringert sich zuerst dessen Volumen, da die Luft zwischen den Partikeln verdrängt wird, siehe Fig. 1(b). Diese Phase wird "Umpackphase" genannt, und ist beschränkt durch das Erreichen der höchstmöglichen Packungsdichte und/oder durch Reibung an Kontaktpunkten der PulverPartikel. Danach werden die meisten Materialien bis zur Plastizitätsgrenze elastisch deformiert, siehe Fig. 1(c). Diese Phase nennt man "Quetschphase". Durch die Volumenreduktion können die Partikel auch Sprödbrüche erleiden. Daran anschließend können die Komponenten plastisch und/oder viskoelastisch deformiert werden.

Die diffraktive Mikrostruktur wird hauptsächlich durch diese plastische bzw. viskoelastische Deformation in die Tablettenoberfläche eingebracht. Viele zum Tablettenpressen verwendete Materialien, wie z.B. einige als Bindemittel verwendete Polymere, zeigen viskoelastisches Verhalten. Wird die Oberfläche der Partikel mit einem plastischen Material beschichtet, kann die Plastizität eines Pulvers weiter verbessert werden. Partikel können mit einem Binder wie beispielsweise Polyvinylpyrrolidon (PVP) teilbeschichtet werden, z.B. in feuchter Granulation, wodurch die Kompressibilität der Partikel verbessert wird. Aufgrund von Partikel-Partikel-Wechselwirkungen wird die mechanische Widerstandskraft der Tablettierungsmasse immer stärker, je größer die angewandte Presskraft ist. Bei Partikel-Partikel-Wechselwirkungen werden an den Partikeloberflächen Bindungen gebildet, da die Anzahl Berührungspunkte zunimmt. Je nach chemischer Zusammensetzung sind die Bindungen ionische oder kovalente Bindungen, Dipol-Dipol-Wechselwirkungen und van-der-Waals Kräfte. Oft ist eine Mischung dieser Bindungen vorhanden. Zusätzlich kann es zu einer Verfestigung flüssiger Filme kommen. Die Verfestigung flüssiger Filme kann auf zwei Arten erfolgen. Erstens, wenn Reibungswärme an den Berührungspunkten dazu führt, dass ein Inhaltsstoff mit tiefem Schmelzpunkt erweicht oder schmilzt, wodurch die mechanische Spannung an dieser Stelle abgebaut wird. Der Inhaltsstoff verhärtet sich dann über eine Schmelzverbindung wieder. Zweitens kann sich ein Inhaltsstoff an Berührungspunkten mit hoher Spannung in dem an der Oberfläche eines Partikels vorhandenen Flüssigkeitsfilm lösen. Auch hier wird die mechanische Spannung abgebaut und das Material rekristallisiert sich, um eine Bindung zu bilden. Erfolgt die Verhärtung nahe der Oberfläche des mikrostrukturierten Presswerkzeugs, unterstützt der erweichte, geschmolzene oder gelöste Inhaltsstoff die Replikation der diffraktiven Mikrostruktur.

Am Ende des Tablettenpressprozesses wird der Druck weggenommen, Fig. 1(d), und die fertige Tablette 4 ausgeworfen, Fig. 1(e). Die anschließende elastische Rückformung muss gering gehalten werden, um eine hohe mechanische Stabilität der Tablette zu erreichen. Dazu wird die Rezeptur entsprechend optimiert.

Für Tabletten mit diffraktiven Mikrostrukturen in ihren Oberflächen bedarf es also einer Rezeptur, welche alle Anforderungen der Tablettenherstellung erfüllt und immer noch eine genügend hohe plastische Deformierbarkeit aufweist, um die Mikrostruktur einbringen zu können. Wie bereits erwähnt, bestehen die zu pressenden Pulver aus einer Mischung verschiedener Substanzen mit unterschiedlichen Funktionen. Der Anteil der plastisch deformierbaren Materialien in der Rezeptur muss so groß wie möglich gewählt sein, wobei jedoch die Anforderungen des Endprodukts wie auch der FDA nach wie vor erfüllt sein müssen. Der Anteil mikrokristalliner Cellulose oder plastischer Bindemittel wie PVP kann z.B. vergrößert werden, oder diese Materialien werden anstelle von äquivalenten, jedoch plastisch weniger deformierbaren Hilfsstoffen verwendet.

Moderne industrielle Tablettenpressen sind Hochleistungs-Maschinen, die Tabletten bei sehr hohen Geschwindigkeiten herstellen können. Die Produktionsgeschwindigkeit modernster Einfachrotationspressen beträgt ungefähr 30'000 bis 300'000 Tabletten pro Stunde. Darüber hinaus müssen sie eine extreme Zuverlässigkeit und Genauigkeit bieten, da alle Tabletten strengen Spezifikationen bezüglich Dicke, Gewicht, Härte und Form erfüllen müssen. Die Maschinen sowie alle ihre Bestandteile müssen GMP (Good Manufacturing Process) und FDA-konform sein.

In Tabelle 3 sind Beispiele für geschwindigkeitsspezifische Daten für verschiedene Tablettenpressen aufgeführt. Weitere Ausführungen findet man in N. A. Armstrong, "Considerations of Compression Speed in Tablet Manufacture", Pharmaceutical Technology, September 1990, S. 106-114. Die kurze Presszeit genügt, um das pulverförmige Rohmaterial in eine harte Tablette zu pressen.

**Tabelle 3**

| Typ der Presse | Geschwindigkeit pro Pressform | Absenkzeit für die letzten 5 mm | Haltezeit mit |
|---|---|---|---|
| Exzentrisch | 85 Tabletten/min. | 68.6 ms | 0 ms |
| Kleine Rotationspresse | 44 Tabletten/min. | 61.4 ms | 10.84 ms |
| Grosse Rotationspresse | 100 Tabletten/min. | 26.7 ms | 3.94 ms |
| Grosse Rotationspresse | 121 Tabletten/min. | 19.1 ms | 3.16 ms |

Die Absenkzeit plus die Haltezeit ist etwa gleich oder etwas weniger lang wie die Zeit, um in Roll-to-Roll-Prozessen (R2R) diffraktive Mikrostrukturen in Polymerfolien heisszuprägen. Solche R2R-Prozesse werden z.B. zur Herstellung von Hologrammen für Banknotensicherheit eingesetzt und arbeiten mit Polymer-Zuführgeschwindigkeiten von ca. 100 m/min. Das Polymersubstrat, die Prozessparameter sowie die Temperatur werden für eine gute Replikation der Mikrostruktur optimiert.

Analog dazu wird der Pressprozess im erfindungsgemäßen Verfahren an die Anforderungen der Mikrostrukturierung angepasst. Die meisten pharmazeutischen Pillen haben eine runde Form. Dies erleichtert den Produktionsprozess, da das Presswerkzeug rotationssymmetrisch ist und während des Pressprozesses frei rotieren kann. Für die Einbringung der diffraktiven Mikrostruktur ist es jedoch von Vorteil, wenn eine Rotation der Pressstempel verhindert wird, um die auftretenden Scherkräfte, insbesondere während der Ablösung des Werkzeugs von der Tablette, zu vermindern, denn während sich die Pressstempel von der Oberfläche der Tabletten wegbewegen, können aufgrund elastischer Rückformung die Tablette und die Werkzeugoberflächen für kurze Zeit in Kontakt bleiben.

_Schutz der mikrostrukturierten Tablettenoberfläche vor mechanischer Beschädigung_

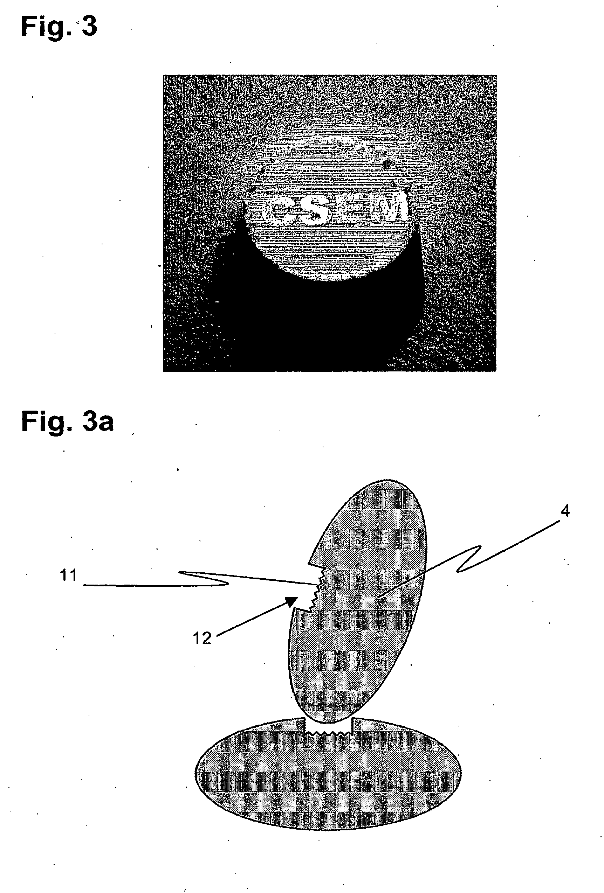

Zum Schutz der Mikrostruktur während des ganzen Produktlebenszyklus vor mechanischen Einwirkungen, insbesondere vor abrasiven Kräften, kann beispielsweise der Kontakt der mikrostrukturierten Fläche mit andern Flächen minimiert werden, indem die diffraktive Mikrostruktur 11 in eine makroskopische Vertiefung 12 in der Oberfläche der Tablette 4 angeordnet wird (siehe Figur 3a).

Solche makroskopischen Vertiefungen 12 sind beim herkömmlichen direkten Tablettierungsprozess üblich. Sie werden hauptsächlich für Marketingzwecke gebraucht, um z.B. das Logo des Unternehmens etc. zu zeigen. Ist die Vertiefung 12 tief und klein genug, so dass die schärfste Kante einer anderen Pille die mikrostrukturierte Fläche nicht berühren kann (siehe Figur 3a), ist die diffraktive Struktur vor mechanischen Beschädigungen gut geschützt. Auch in Sammelbehältern, Sortiermaschinen oder in Aufbewahrungsflaschen kann keine Abrasion stattfinden. Weniger tiefe Vertiefungen bieten einen weniger guten Schutz, sind zuweilen jedoch aufgrund von Designanforderungen unumgänglich.

Die mikrostrukturierten Tabletten können alternativ oder zusätzlich auch mit einer zusätzlichen Schutzschicht beschichtet werden, ohne den diffraktiven Effekt zu zerstören, vorausgesetzt, die Schutzschicht ist im sichtbaren Spektralbereich transparent und hat einen Brechungsindex, der nicht demjenigen des Materials entspricht, das die Mikrostruktur trägt. Eine solche Beschichtung schützt die diffraktive Mikrostruktur ebenfalls. Ist der Brechungsindex dieser Beschichtung höher, die Dicke unterhalb von 1 $\mu$m und die Gitterperiode der Mikrostruktur unter 500 nm, dann können diffraktive Farbeffekte nullter Ordnung realisiert werden. Diese Farbeffekte sind äußerst fälschungssicher und leicht zu erkennen.

_Beispiel einer erfindungsgemäßen pharmazeutische Tablette mit einer diffraktiven Mikrostruktur_

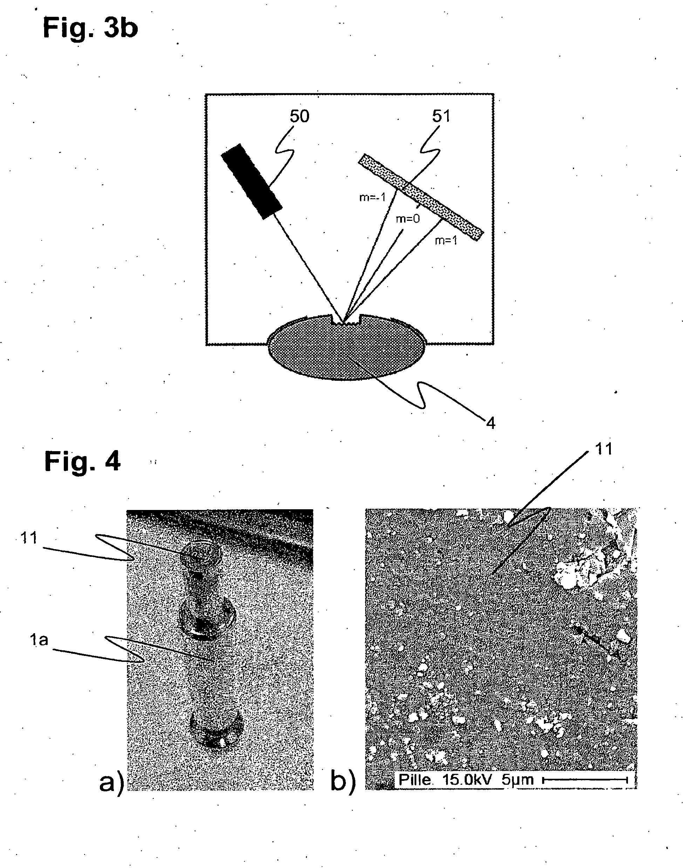

Ein gemäß Tabelle 1 zusammengesetztes Pulvergemisch wurde in einer Einfachrotationspresse des Typs 1200i der Firma Fette, Deutschland, mit 24 Pressstempelpaaren zu Tabletten komprimiert. Die Pressstempel hatten einen Durchmesser von 11,8 mm und eine hartverchromte Oberfläche. In die hartverchromte Oberfläche wurde eine diffraktive Mikrostruktur mit einer Periode von 1.4 $\mu$m und einer Tiefe von ca. 500 nm ionengeätzt, siehe Fig. 4(ä). Sichtbare diffraktive Effekte in Tabletten mit einem Gewicht von 540 mg wurden mit einer Presskraft von 25 kN und einer Produktionsgeschwindigkeit von 30'000 Tabletten pro Stunde erreicht. Figur 3 zeigt eine der hergestellten Tabletten. Die diffraktive Mikrostruktur erzeugt einen eindeutig sichtbaren Schriftzug "CSEM". Die erzeugten Beugungseffekte können natürlich mit der schwarzweißen Aufnahme in Fig. 3 nicht dargestellt werden. Die Härte der Tablette liegt bei 154 N, was in Bezug auf die Auflösbarkeit der Tablette ein zufrieden stellender Wert ist. Figur 4(b) zeigt ein SEM-Bild der mikrostrukturierten Oberfläche einer solchen Tablette. Die diffraktive Mikrostruktur ist deutlich sichtbar.

_Authentifizierung von emhdungsgemäßen Tabletten_

Falls erfindungsgemäße Tabletten eine helle und/oder leuchtende Farbe aufweisen, kann dieser starke Hintergrund das Erkennen eines Regenbogeneffekts der diffraktiven Mikrostrukturen erschweren. Da die üblichen Pulverkomponenten im sichtbaren Spektralbereich einen Brechungsindex von ungefähr 1,5 haben, wird nur ein kleiner Prozentsatz des auf die Tablettenoberfläche einfallenden Lichts in die erste oder höhere Beugungsordnungen zurückgebeugt. Die Winkelverteilung des gebeugten Lichts ist gegeben durch:

$$\Lambda(\sin \theta_m - \sin \theta_i) = m\lambda$$

wobei $\theta_m$ der Reflexionswinkel der m-ten Beugungsordnung, $\theta_i$ der Einfallswinkel und $\lambda$ die Wellenlänge des Lichts ist (siehe Figur 2(a)).

Da Beugungseffekte höherer Ordnung schwächer sind, ist ein Erkennen des typischen Beugungsmusters für einen Laien eventuell nicht ganz einfach. Die tiefe reflektierte Intensität ist jedoch kein Nachteil, da starke diffraktive Farbeffekte den Endverbraucher irritieren könnten. Viele Patienten schrecken vor stark gefärbten Pillen zurück. Andererseits kann die Sichtbarkeit des Beugungs-Effekts leicht durch eine geeignete Beleuchtung bei einem optimierten Einfallswinkel erhöht werden. Dies macht den Effekt zu einem so genannten Sicherheitsmerkmal zweiter Stufe. In der Arzneimittelindustrie sind Sicherheitsmerkmale zweiter oder dritter Stufe weit verbreitet, da die Unternehmen ihren Endverbrauchern nicht unbedingt zu erkennen geben wollen, dass Fälschungen ein Problem darstellen. Unter Beleuchtung mit beispielsweise einer weißen LED leuchtet der Regenbogeneffekt der diffraktiven Mikrostruktur bei einem gewissen Betrachtungswinkel auf. Eine geübte Person kann mit Hilfe eines solchen Verifizierungsgeräts das Vorhandensein der diffraktiven Mikrostruktur in weniger als einer Sekunde überprüfen.

Die Überprüfung des Vorhandenseins einer diffraktiven Mikrostruktur ist eine qualitative Authentifizierung. Eine einfache und schnelle Methode zur quantitativen Prüfung diffraktiver Mikrostrukturen besteht darin, die Strukturen mit dem Strahl aus einer Laserdiode (z.B. $\lambda$=650 nm) in einem festgelegten Einfallswinkel zu beleuchten. Der Laserstrahl wird gemäß der oben angegebenen Formel in die verschiedenen Beugungsordnungen gebeugt. Da die Laserwellenlänge $\lambda$ sowie der Einfallswinkel $\theta_i$ bekannt sind, kann die Periode A der Mikrostruktur ermittelt werden, indem der Beugungswinkel mindestens einer Ordnung gemessen wird. Dies geschieht z.B. mit Hilfe eines tragbaren Lesegeräts, welches eine Vertiefung aufweist, in der die Pille fixiert wird und die einen festgelegten Einfallswinkel des Laserstrahls gewährleistet (siehe Figur 3b). Die gebeugten Laserstrahlen werden durch ein Array von Photodioden aufgefangen, und die Periode der Mikrostruktur wird aufgrund der Positionen der gebeugten Strahlen berechnet. Solche mobilen Lesegeräte können beispielsweise in Apotheken oder durch Zollbehörden eingesetzt werden.

*Herstellen eines erfindungsgemäßen Presswerkzeugs*

Das Material des Werkzeugs, das die Mikrostruktur trägt, muss sehr hart sein, um eine lange Lebensdauer zu garantieren. Gleichzeitig muss es jedoch möglich sein, die Mikrostruktur in seine Oberfläche einzubringen. Geeignete Materialien sind z.B. gehärteter Stahl, hartverchromter Stahl, Wolframkarbid oder Molybdänkarbid. All diese Materialien sind von der FDA genehmigt und können für die Pressstempel oder die Pressformen verwendet werden. Diese Materialien sind jedoch mit den herkömmlichen holographischen und lithographischen Techniken nicht kompatibel. Sie können jedoch mit anderen Verfahren mikrostrukturiert werden, die nachfolgend beschrieben werden.

*Ionenätzen*

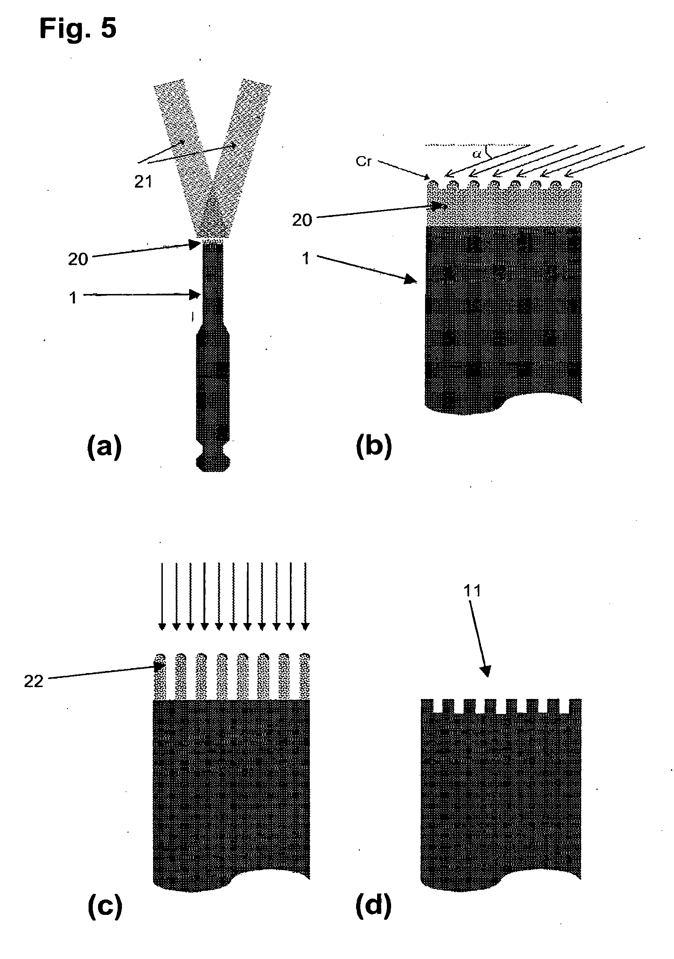

Gehärteter Stahl, Stahl mit Hartchrombeschichtung, Wolframkarbid oder Molybdänkarbid kann mit einer speziellen Ionenätztechnik mikrostrukturiert werden. Diese Technik umfasst folgende Schritte, welche in Figur 5 (a) bis (d) schematisch dargestellt sind:

1. Eine dünne, lichtempfindliche Schicht 20, ein so genannter Photoresist, wird auf die Oberfläche des Presswerkzeuges aufgetragen, welche mikrostrukturiert wird. In Figur 5(a) ist dies ein Pressstempel 1. Die Beschichtung wird in einem speziellen Raum ohne Blau- und UV-Strahlung vorgenommen. Geeignete Photoresist-Materialien sind z.B. ma-N440 (MRT) Microposit S1800 (Röhm & Haas) und AZ1500 (Clariant). Die optimale Dicke der Schicht 20 liegt im Bereich von 300 nm bis 2000 nm. Die Beschichtung kann, falls das Werkzeug angemessen fixiert wird, durch Spin-Coaten (Convac 1001s) oder durch Spray-Coaten (EFD MicroCoat MC780S) erfolgen. Letzteres muss für eine gute Homogenität im gewünschten Dickebereich optimiert sein. Nach der Beschichtung wird bei 100 bis 120°C während 1 - 60 min (je nach Dicke und Material der Schicht) gehärtet (sog. Soft Bake).

2. Als nächstes wird die Photoresist-Schicht 20 in einem holographischen Belichtungs-Aufbau mit zwei interferierenden Laserstrahlen 21 belichtet (siehe Figur 5(a)). Gekreuzte Gitter werden durch zwei orthogonale Belichtungen realisiert. Die integrierte Leistung wird durch eine Photodiode kontrolliert und hängt vom Photoresist-Material und den gewünschten Gitterparametern ab. Der Laser ist beispielsweise ein HeCd-Laser mit einer Wellenlänge $\lambda$=441.6 nm. Je nach Einfallswinkel $\Theta$ der beiden Strahlen sowie den optischen Komponenten des verwendeten Holographie-Aufbaus sind Gitterperioden A von 270 nm bis zu 16'000 nm möglich, $A = \lambda / (2n \sin \Theta)$, n ist der Brechungsindex des Materials, durch welches die Laserstrahlen die Photoresistoberfläche beleuchten. Findet die Beleuchtung in Luft statt, ist n=1. Um die Form der Gitterfläche zu definieren, können Schattenmasken benutzt werden. Auf diese Weise können z.B. Logos, Markennamen etc. realisiert werden.

3. Nach der Belichtung wird die Photoresist-Schicht in einer geeigneten Entwicklungslösung entwickelt. Dafür können z.B. der Grundentwickler S303 (Microposit) oder Konzentrat (Microposit) verwendet werden. Die Entwicklungszeit hängt von den herzustellenden Gitterparametern ab. Sofort nach der Entwicklung wird das Werkzeug in ein Stoppbad mit reinem Wasser gelegt. Die Temperaturen beider Bäder liegen bei 30°C und werden auf $\pm0.2$°C kontrolliert. Am Ende des Entwicklungsschrittes verfügt die Photoresist-Schicht auf dem Pillenpresswerkzeug über ein Gitter mit der gewünschten Periode und Tiefe (siehe Figur 5(b)). Die Form kann wie gezeigt sinusförmig oder auch komplexer sein.

4. Um das Gitter in der Werkzeugoberfläche trockenätzen zu können, muss ein Kontrast in der Ätzrate von mindestens 2:1 realisiert werden. Dies wird erreicht, indem eine Metallhaube, vorzugsweise eine Chromhaube, mit einer Massendicke von 10 nm bis 200 nm auf die erhöhten Stellen des Gitters auf der Photoresist-Schicht 20 aufgetragen wird. Die optimale Dicke hängt von der Gittertiefe und -periode ab. Das Tablettenpresswerkzeug mit der entwickelten Photoresist-Schicht 20 wird so in einer Vakuumkammer (Balzers BAK550) angeordnet, dass die aufgedampften Atome die Vertiefungen des Gitters nicht erreichen können. Diese Schiefbedampfung wird in Figur 5(b) schematisch dargestellt. Der Einfallswinkel $\alpha$ der Metallatome liegt dabei je nach Gittertiefe und -periode zwischen 3° bis 45°. Falls nötig wird die Schiefbedampfung von zwei oder mehr Seiten her vorgenommen, um symmetrische Metallkappen zu erhalten.

5. Nun wird die Photoresist-Schicht 20 geöffnet, womit eine Maske 22 resultiert. Wie in Figur 5(c) gezeigt, werden die Teile des Polymer-Resistmaterials ohne Chromkappen mit $O_2$-Plasma (Oxford-RIE) geätzt. Die kinetische Energie der reaktiven Sauerstoffionen liegt im Bereich von 500 eV. Die Ätzrate hängt darüber hinaus vom Druck in der Vakuumkammer ab. Das Ende dieses Öffnungsschrittes wird durch ein Endpunktdetektionssystem festgestellt, welches auf Laser-Interferometrie basiert.

6. Die geöffnete Maske 22 wird anschliessend dazu benutzt, die Gitterstruktur 11 durch einen weiteren Trockenätzschritt in die Werkzeugoberfläche zu transferieren. Diese Ätzung in die harte Oberfläche des Pillenpresswerkzeugs erfolgt durch Beschießung mit Argon-Ionen (Veeco RF 350) mit einer kinetischen Energie in der Größenordnung von 500 eV. Bei 500 eV ist die Energie niedrig genug, um eine hohe Eindringtiefe der Quellionen in die Probe zu verhindern, ohne jedoch die Ätzrate zu vermindern. In Tabelle 4 werden für verschiedene Elemente und Verbindungen typische Ätzraten r für eine solche Argon-Beschießung bei einer Ionenstromdichte von 1 mA/cm$^2$, einer kinetischen Energie der Ionen von 500 eV und bei senkrechtem Beschuss aufgelistet.

**Tabelle 4**

| Element oder Verbindung | Argon-Ätzrate r [nm/min] |
|---|---|
| Al | 73 |
| C | 4.4 |
| Cr | 58 |
| Cu | 110 |
| Fe | 53 |
| Mo | 54 |
| Ni | 66 |
| Si | 38 |
| SiC | 35 |
| $SiO_2$ | 40 |
| Ta | 42 |
| TaC | 10 |
| Ti | 38 |
| V | 37 |
| W | 38 |
| Zr | 62 |

Ist die gewünschte Gittertiefe erreicht, wird das restliche Chrom und Photoresist-Material entfernt und zurück bleibt die fertige, mikrostrukturierte Oberfläche des erfindungsgemäßen Presswerkzeugs (siehe Figur 5(d)).

Für eine kostengünstige Herstellung der diffraktiven Mikrostrukturen auf den erfindungsgemäßen Presswerkzeugen werden bei den zeitaufwendigsten Schritten, der Schiefbedampfung und Trockenätzung, mehrere solche Werkzeuge parallel hergestellt.

Mit der genannten Ionenätzmethode lassen sich auch beschichtete Presswerkzeuge mikrostrukturieren, wie beispielsweise galvanische Hartchrombeschichtungen. Figur 4(a) zeigt ein Bild eines erfindungsgemäßen Pressstempels 1a mit einer galvanisch abgeschiedenen Hartchromoberfläche. In die Oberfläche wurde gemäß der oben beschriebenen Methode eine diffraktive Mikrostruktur 11 eingebracht. Figur 4(b) zeigt ein SEM-Bild der mikrostrukturierten Oberfläche einer mit diesem Presswerkzeug gepressten Tablette.

*Prägung*

Eine weitere Methode zur Einbringung einer diffraktiven Mikrostruktur auf einen erfindungsgemäßen Pressstempel besteht darin, die gewünschte Mikrostruktur durch ein Prägungsverfahren mit Hilfe eines Hauptwerkzeugs in die Oberfläche der erfindungsgemäßen Presswerkzeuge einzuhämmern. Dieses Hauptwerkzeug kann mit der obenstehend beschriebenen Ionenätzmethode mikrostrukturiert werden.

Es ist bekannt, makroskopische Strukturen, wie z.B. Fahrgestellnummer oder Markennamen, in Metall einzuhämmern. Solche Strukturen sind im kleinsten Fall typischerweise einige Millimeter gross. Die benötigte Genauigkeit der Strukturierung ist gering, da die einzige Anforderung darin besteht, die Zahlen und Buchstaben lesen zu können. Das Einhämmern diffraktiver Mikrostrukturen mit Perioden in der Größenordnung von 1 µm in erfindungsgemäßen Presswerkzeugen ist natürlich erheblich komplizierter. Die erforderliche Genauigkeit ist sehr hoch, um den Interferenzeffekt der Mikrostrukturen zu erhalten. Darüber hinaus sind die Mikrostrukturen kleiner als die inneren Strukturen von Metallen (Körnergröße), und die Werkzeuge sind aus sehr harten Metalllegierungen gefertigt.

Für ein leichteres Verständnis dieser Methode werden nachfolgend einige charakteristische mechanische Eigenschaften von Metallen zusammengefasst. Metalle neigen dazu, wegen der Stärke der metallischen Bindung hohe Schmelzpunkte zu haben. Die Bindungsstärke ist von Metall zu Metall verschieden und hängt unter anderem von der Anzahl Elektronen ab, die jedes Atom in das sogenannte freie Elektronengas abgibt. Darüber hinaus hängt sie von der Packungsdichte ab. Jedes Metall besteht aus einer Vielzahl von einzelnen Körnern bzw. Kristalliten, also perfekt geordneten mikrokristallinen Gebieten. Der durchschnittliche Durchmesser solcher Körner liegt typischerweise zwischen 10 µm und 100 µm. An den Korngrenzen, auch Dislokationen genannt, sind die Atome falsch ausgerichtet. Spezialbehandlungen ermöglichen kleinere Korngrößen und somit härtere Metalle.

Wirkt auf ein Metall eine kleine mechanische Spannung, beginnen einzelne Metalllagen übereinander zu gleiten. Sobald die Spannung abgebaut wird, fallen die Atome wieder zurück in ihre Ursprungsposition (elastische Verformung). Ist die Spannung größer, gleiten die Atome in eine neue Position; das Metall ist dauerhaft verformt (plastische Deformation). Durch die Bewegung der Dislokationen führt sie zur Aufbrechung einer beschränkten Anzahl atomarer Bindungen. Die Kraft, die dazu benötigt wird, die Bindungen aller Atome in einer Kristallebene gleichzeitig aufzubrechen, ist sehr groß ist. Die Bewegung der Dislokationen erlaubt es Atomen in Kristallebenen jedoch, bei viel geringeren Spannungen aneinander vorbeizugleiten. Da die zur Bewegung benötigte Energie entlang der dichtesten Kristallebenen am geringsten ist, haben die Dislokationen innerhalb eines Metallkornes eine bevorzugte Bewegungsrichtung. Dies führt zu Gleitverschiebungen entlang paralleler Ebenen innerhalb des Kornes. Der Durchmesser solcher Gleitlinien liegt typischerweise im Bereich von 10 nm bis 1000 nm. Diese Gleitlinien gruppieren sich und formen Gleitlinienstreifen. Letztere sind unter einem optischen Mikroskop bereits sichtbar. Wie untenstehend beschrieben werden wird, unterstützen die Gleitlinien und Gleitlinienstreifen die Replikation von Mikrostrukturen. Die Verschiebung der Atomlagen übereinander wird durch Korngrenzen behindert, die auf eine unpassende Konstellation der Atomreihen zurückzuführen sind. Das bedeutet, dass je mehr Korngrenzen ein Metallstück aufweist, d.h. je kleiner die einzelnen Kristallkörner sind, desto härter ist das Metall. Da die Korngrenzen Gebiete sind, wo die Atome keinen guten Kontakt zu einander haben, neigen Metalle dazu, an Korngrenzen zu brechen. Somit wird das Metall durch eine Erhöhung der Anzahl Korngrenzen nicht nur härter, sondern auch brüchiger. Je härter ein Metall ist, desto schwieriger ist es zu verformen. In Tabelle 5 sind Vickers Härtegrade (HV), die Materialdichte ρ sowie der Elastizitätsmodul oder Young'sche Modul E für verschiedene Materialien (nicht nur Metalle und Legierungen) aufgeführt.

**Tabelle 5**

| Material | $\rho$ [g/cm$^3$] | Härtegrad [HV] | E [GPa] | $\sigma_y$ [MPa] | $\sigma_u$ [MPa] |
|---|---|---|---|---|---|
| Diamant (C) | 3.52 | 10060 | 1000 | - | - |
| Polykristalliner Diamant / diamantenähnlicher Kohlenstoff DLC (C) | 2.8-4.1 | 3000 - 12000 | 150-800 | - | - |
| Kubisches Bornitrid (c-BN) | 3.48 | 4500 | 680 | - | 50 |
| Siliziumcarbid (SiC) | 3.22 | 3300 | 480 | - | 140 |
| Borcarbid (B$_4$C) | 2.5 | 3200 | 450 | - | 380 |
| Titancarbid (TiC) | 4.93 | 3200 | 460 | - | 330 |
| Vanadiumcarbid (VC) | 5.4 | 2940 | 420 | - | - |
| Aluminiumnitrid (AlN) | 3.2 | 2500 | 350 | - | 500 |
| Wolframcarbid (W$_2$C) | 15.6 | 2400 | ≈700 | - | 530 |
| Titannitrid (TiN) | 5.22 | 2100 | 260 | - | - |
| Korund (Al$_2$O$_3$) | 3.97 | 2060 | ≈400 | - | 320 |
| Molybdäncarbid (Mo$_2$C) | 8.2 | 1950 | 550 | - | - |
| Tantalcarbid (TaC) | 13.9 | 1800 | ≈340 | - | - |
| Siliziumnitrid (Si$_3$N$_4$) | 3.2 | 1400 - 1700 | ≈340 | - | 580 |

(fortgesetzt)

| Material | $\rho$ [g/cm$^3$] | Härtegrad [HV] | E [GPa] | $\sigma_y$ [MPa] | $\sigma_u$ [MPa] |
|---|---|---|---|---|---|
| Zirkonoxid (ZrO$_2$) | 5.6 | 1400 - 1600 | 240 | - | 1000 |
| Chrom (Cr) | 6.9-7.2 | 750 - 1050 | 289 | 360 | 690 |
| Gehärteter Nickel | 7.9-8.1 | 600 - 950 | 214 | - | - |
| Gehärteter Stahl | ≈7.8 | 500 - 900 | 190 - 214 | 520 | 860 |
| Nickel | 8.91 | 550 | 214 | 940 | 1010 |
| Ungehärteter Stahl | ≈7.8 | 100 - 500 | 190-214 | 365 | 900 |
| Aluminium | 2.7 | 25 | ≈70 | 260 | 290 |

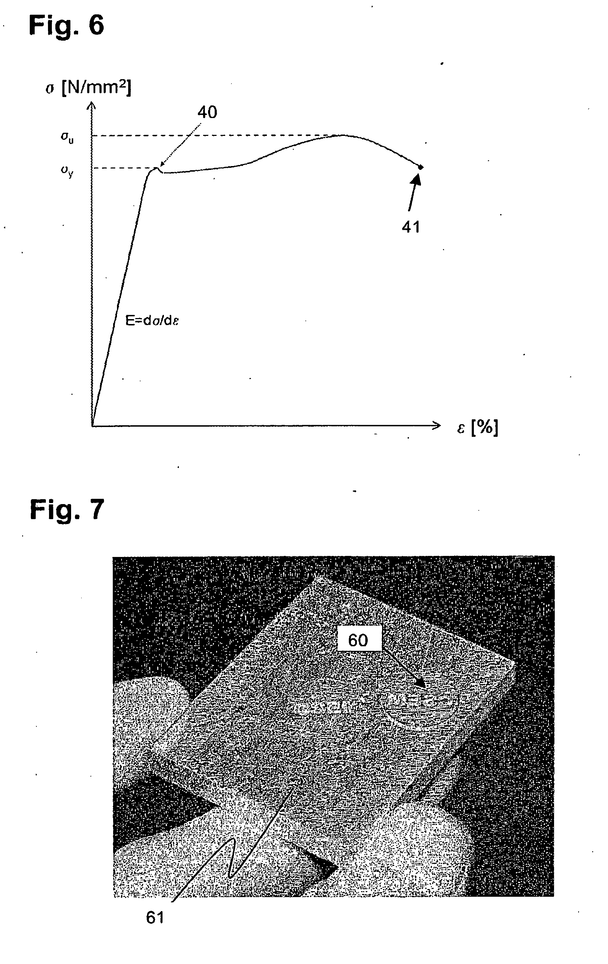

Der Elastizitätsmodul ist unabhängig vom Härtegrad. Der Härtegrad ist ein Maß, bei dem die plastische Deformation durch mechanische Spannung beginnt. Der Young'sche Modul E=d$\sigma$/d$\epsilon$ ist die Steigung des linearen Teils der Spannungs-/Dehnungskurve $\sigma(\epsilon)$. Figur 6 zeigt ein Beispiel einer solchen Kurve für ein duktiles Material wie beispielsweise Stahl. Je grösser der Widerstand eines Materials gegen elastische Deformation ist, desto grösser ist der Wert des Young'schen Moduls. Über der elastischen Grenze (40) erfolgt die plastische Deformation. Die Streckgrenze $\sigma_y$ misst den Widerstand zur plastischen Deformation. Jede Spännungserhöhung über die Streckgrenze (40) hinaus verursacht eine dauerhafte Verformung des Materials. In dieser so genannten Fliesszone ist die Deformation selbst bei kleinen Spannungserhöhungen relativ groß. Diesen durch eine sehr kleine Steigung der Spannungs-/Dehnungskurve charakterisierten Prozess bezeichnet man oft als "perfekte Plastizität". Nach dem Fliessen wird die Spannung bis zur Bruchfestigkeit oder ultimativen Zugspannung $\sigma_u$ erhöht, bei der das Material bricht (41). Bei brüchigen Materialien existiert die Fliesszone praktisch gar nicht. Brüchige Materialien haben im Vergleich zu duktilen Materialien oft relativ hohe Young'sche Module und ultimative Zugspannungen. In Tabelle 5 sind Maximalwerte für $\sigma_y$ bzw. $\sigma_u$ aufgeführt. Alle Werte in Tabelle 5 sind lediglich Referenzwerte. Die Daten von richtigen Proben können erheblich davon abweichen. Insbesondere Werte von Beschichtungen solcher Materialien hängen unter anderem von den Prozessparametern und vom Wachstumsmechanismus ab.

Um die diffraktive Mikrostruktur mit einem Hauptwerkzeug in ein erfindungsgemäßes Presswerkzeug einzuhämmern zu können, müssen folgende Voraussetzungen erfüllt sein:

1. Die Härte des Hauptwerkzeugs muss größer sein als diejenige des Presswerkzeugs.
2. Der Young'sche Modul muss für beide so hoch wie möglich sein, um die elastische Deformation zu minimieren.
3. Die angewandte Spannung muss höher als die Streckgrenze, jedoch tiefer als die ultimative Zugspannung des Presswerkzeugs sein. Außerdem muss sie niedriger sein als die Streckgrenze (falls vorhanden) und die ultimative Zugspannung des Hauptwerkzeugs.

Gegebenenfalls kann das Presswerkzeug oder dessen Oberfläche nach dem Einhämmern der Mikrostruktur durch eine anschließende Wärmebehandlung oder Ionen-Implantation gehärtet werden.

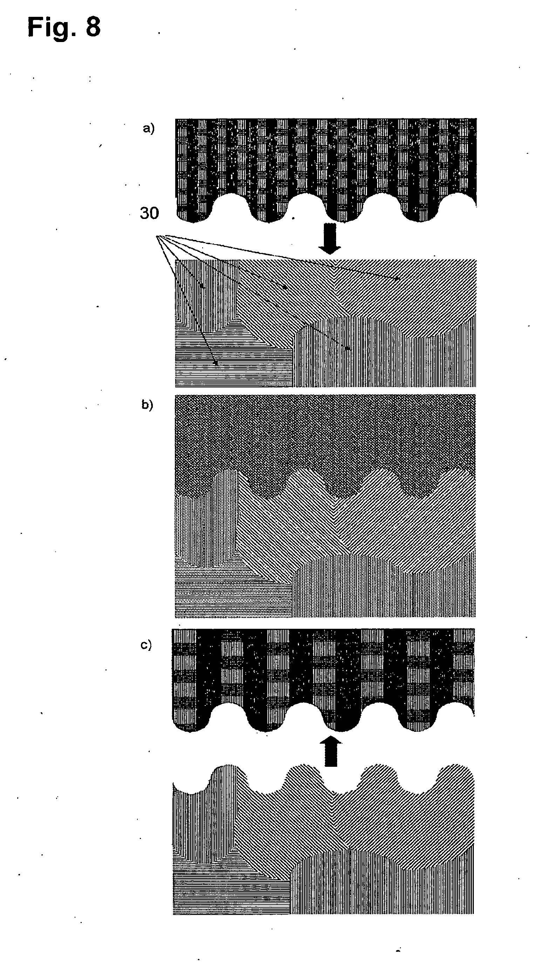

Figur 8 zeigt schematisch auf, wie die Mikrostrukturen des Hauptwerkzeugs beim Prägeschritt durch das Füllen der Hohlräume durch die Gleitebenen in den Metallkörnern auf das Presswerkzeug repliziert werden.

Um ein Presswerkzeug mit einer galvanisch hartverchromten Oberfläche mikrostrukturieren zu können, ist beispielsweise ein Hauptwerkzeug aus Wolframkärbid notwendig, und eine Prägekraft von ca. 400 - 500 MPa. Alternativ dazu kann das Hauptwerkzeug auch aus gehärtetem Stahl bestehen, mit einer Beschichtung z.B. aus Wolframkarbid, Si$_3$N$_4$ oder ZrO$_2$, welche die Mikrostruktur trägt. Letztere Variante ist kostengünstiger, da nur die Beschichtung aus dem sehr harten und bruchresistenten Material bestehen muss. Figur 7 zeigt ein Beispiel einer derart mit einem Prägeverfahren hergestellten Mikrostruktur auf einer Metalloberfläche. Ein Block Aluminium 61 mit einer Dicke von ca. 4 mm wurde mit einem runden Nickel-Shim 60 mit einem Durchmesser von ca. 12 mm mikrostrukturiert. Der Nickel-Shim 60.liegt in Figur 7 auf dem Metallblock 61. Der Shim weist ein diffraktives Gitter mit einer Periode von 1400 nm und einer Tiefe von ungefähr 300 nm auf, welches spiegelverkehrt die vier Buchstaben CSEM zeigt. Dieser Shim wurde bei Raumtemperatur mit einem Druck von 3 Tonnen während ca. 0,5 Sek. auf den Aluminiumblock gedrückt. Wie aus Figur 7 ersichtlich ist, wurde die diffraktive Mikrostruktur gut auf dem Aluminiumblock nachgebildet.

**Bezugszeichenliste**

| 1, 1a, 1b | Pressstempel |
|---|---|
| 2 | Pulvermischung |
| 3 | Pressform |
| 4 | Tablette |
| 10 | Gitterlinie |
| 11 | Gitter-Mikrostruktur |
| 12 | Vertiefung |
| 20 | Photoresist-Schicht |
| 21 | Interferierende Laserstrahlung |
| 22 | Maske |
| 30 | Körner, Kristallite |
| 40 | Elastische Grenze |
| 41 | Bruch |
| 50 | Laser |
| 51 | Photodioden |
| 60 | Nickel-Shim |
| 61 | Metallblock |

| A | Periode |
|---|---|
| t | Tiefe |
| θm | Reflexionswinkel der m-ten Beugungsordnung |
| θi | Einfallswinkel |
| m | Beugungsordnung |

**Patentansprüche**

1. Tablette (4), insbesondere zur pharmazeutischen Verwendung, bestehend aus einer Vielzahl von einzelnen Pulverpartikeln,

   **dadurch gekennzeichnet, dass**
   auf mindestens einem Teil der an der Oberfläche der Tablette (4) sich befindenden Pulverpartikel diffraktive Mikrostrukturen (11) mit einer Gitterperiode von kleiner als 500 nm eingeprägt sind, wobei diese mikrostrukturierte Tablette (4) zusätzlich mit einer im sichtbaren Spektralbereich transparenten Schutzschicht beschichtet ist, wobei die Schutzschicht einen Brechungsindex aufweist, der höher ist als der Brechungsindex der mikrostrukturierten Pulverpartikel und die Dicke der Schutzschicht kleiner als 1 μm ist.

2. Tablette nach Anspruch 1, wobei die Gitter-Mikrostruktur (11) ein Relief mit einem im wesentlichen dreieckigen oder sinusförmigen Profil aufweist.

3. Tablette nach einem der vorhergehenden Ansprüche, wobei die Gitter-Mikrostrukturen (11) lineare Gitter oder Lochraster-Gitter sind.

4. Tablette nach einem der vorhergehenden Ansprüche, wobei die Periodenlänge Λ der Gitter-Mikrostruktur (11) zwischen 300 nm und 5000 nm beträgt, bevorzugt zwischen 800 und 2500 nm.

5. Tablette nach einem der vorhergehenden Ansprüche, wobei die Tiefe t des Reliefs zwischen den Gitterlinien (10) der Gitter-Mikrostruktur (11) mindestens 80 nm beträgt, bevorzugt 300 nm, und besonders bevorzugt 400 nm.

6. Tablette nach einem der vorhergenden Ansprüche, wobei die Tiefe t des Reliefs zwischen den Gitterlinien (10) der Gitter-Mikrostruktur (11) maximal 1000 nm beträgt.

7. Tablette nach einem der vorhergehenden Ansprüche, wobei die Tiefe t des Reliefs zwischen den Gitterlinien (10) der Gitter-Mikrostruktur (11) zwischen 0.3 und 0.4 Periodenlängen Λ der Gitter-Mikrostruktur (11) beträgt.

8. Tablette nach einem der vorhergehenden Ansprüche, wobei die Tablette (4) mit einer im sichtbaren Spektralbereich transparenten Beschichtung versehen wird, welche einen zum Tablettenmaterial unterschiedlichen Brechungsindex aufweist, bevorzugt mit einer Differenz von mindestens 0.2.

9. Tablette nach einem der vorhergehenden Ansprüche, wobei die Tablette (4) mindestens eine Vertiefung (12) in ihrer Oberfläche aufweist, in der die Gitter-Mikrostruktur (11) angeordnet ist.

10. Tablette nach einem der vorhergehenden Ansprüche, wobei die Tablette (4) mittels direkter Tablettierung hergestellt ist.

11. Tablette nach einem der vorhergehenden Ansprüche, wobei Pulverpartikel mit < 75 $\mu$m Durchmesser einen Anteil von 15-25 % des Gesamtgewichts der Pulverpartikel ausmachen;
Pulverpartikel mit 75-150 $\mu$m Durchmesser einen Anteil von 30-50% des Gesamtgewichts der Pulverpartikel ausmachen;
Pulverpartikel mit 150-250 $\mu$m Durchmesser einen Anteil von 15-25% des Gesamtgewichts der Pulverpartikel ausmachen;
Pulverpartikel mit 250-500 $\mu$m Durchmesser einen Anteil von 15-25% des Gesamtgewichts der Pulverpartikel ausmachen; und
Pulverpartikel mit > 500 $\mu$m Durchmesser haben einen Anteil von < 2% des Gesamtgewichts der Pulverpartikel ausmachen.

**Claims**

1. Tablet (4), in particular for pharmaceutical use, comprising a plurality of individual powder particles, **characterized in that** on at least a part of the powder particles situated on surface of the tablet (4) are embossed diffractive microstructures (11) with grating period less than 500mm, wherein this microstructured tablet (4) is additionally coated with a protective coating which is transparent in the visible spectral domain, wherein the protective coating exhibits an index of refraction which is higher than the index of refraction of the microstructured powder particles and the thickness of the protective coating is less than 1 $\mu$m.

2. Tablet according to Claim 1, wherein the grating microstructure (11) comprises a relief with an essentially triangular or sinusoidal profile.

3. Tablet according to one of the preceding claims, wherein the grating microstructures (11) are a linear grating or a hole-raster grating.

4. Tablet according to one of the preceding claims, wherein the period length A of the grating microstructure (11) is between 300 nm and 5000 nm, preferably between 800 nm and 2500 nm.

5. Tablet according to one of the preceding claims, wherein the depth t of the relief between the grating lines (10) of the grating microstructure (11) is at least 80 nm, preferably 300 nm, and especially preferably 400 nm.

6. Tablet according to one of the preceding claims, wherein the depth t of the relief between the grating lines (10) of the grating microstructure (11) is maximum 1000 nm.

7. Tablet according to one of the preceding claims, wherein the depth t of the relief between the grating lines (10) of the grating microstructure (11) amounts to between 0.3 and 0.4 period lengths A of the grating microstructure (11).

8. Tablet according to one of the preceding claims, wherein the tablet (4) is provided with a coating that is transparent in the visible spectral range and has a different refractive index from the tablet material, preferably with a difference of at least 0.2.

9. Tablet according to one of the preceding claims, wherein the tablet (4) has at least one recess (12) in its surface in which the grating microstructure (11) is arranged.

10. The tablet according to one of the preceding claims, wherein the tablet (4) is produced by means of direct tableting.

11. Tablet according to one of the preceding claims,
wherein
powder particles with < 75 $\mu$m diameter make up a portion of 15-25% of the total weight of powder particles;
powder particles with 75-150 $\mu$m diameter make up a portion of 30-50% of the total weight of powder particles;

powder particles with 150-250 µm diameter make up a portion of 15-25% of the total weight of powder particles; powder particles with 250-500 µm diameter make up a portion of 15-25% of the total weight of powder particles; powder particles with > 500 µm diameter make up a portion of < 2% of the total weight of powder particles.

**Revendications**

1. Comprimé (4), en particulier pour utilisation pharmaceutique, constitué d'une pluralité de particules individuelles de poudre, **caractérisé en ce que** sur au moins une partie des particules de poudre se trouvant sur la surface du comprimé (4) sont embossées des microstructures diffractives (11) ayant une période de réseau inférieure à 500 nm, ce comprimé microstructuré (4) étant en outre enrobé d'une couche de protection transparente dans le domaine spectral visible, la couche de protection présentant un indice de réfraction qui est supérieur à l'indice de réfraction des particules de poudre microstructurées, et l'épaisseur de la couche de protection étant inférieure à 1 µm.

2. Comprimé selon la revendication 1, dans lequel la microstructure en réseau (11) présente un relief ayant un profil essentiellement triangulaire ou sinusoïdal.

3. Comprimé selon l'une des revendications précédentes, dans lequel les microstructures en réseau (11) sont des réseaux linéaires ou des réseaux à trame perforée.

4. Comprimé selon l'une des revendications précédentes, dans lequel la longueur de période A de la microstructure en réseau (11) est comprise entre 300 nm et 5000 nm, de préférence entre 800 et 2500 nm.

5. Comprimé selon l'une des revendications précédentes, dans lequel la profondeur t du relief entre les traits (10) de la microstructure en réseau (11) est d'au moins 80 nm, de préférence de 300 nm et d'une manière particulièrement préférée de 400 nm.

6. Comprimé selon l'une des revendications précédentes, dans lequel la profondeur t du relief entre les traits (10) de la microstructure en réseau (11) est au maximum de 1000 nm.

7. Comprimé selon l'une des revendications précédentes, dans lequel la profondeur t du relief entre les traits (10) de la microstructure en réseau (11) est comprise entre 0,3 et 0,4 fois la constante A de la microstructure en réseau (11).

8. Comprimé selon l'une des revendications précédentes, le comprimé (4) étant pourvu d'un revêtement transparent dans le domaine spectral visible, revêtement qui présente un indice de réfraction différent de celui du matériau du comprimé, la différence étant de préférence d'au moins 0,2.

9. Comprimé selon l'une des revendications précédentes, le comprimé (4) présentant au moins un renfoncement (12) dans sa surface, dans lequel est disposée la microstructure en réseau (11).

10. Comprimé selon l'une des revendications précédentes, le comprimé (4) étant fabriqué par comprimage direct.

11. Comprimé selon l'une des revendications précédentes, dans lequel
des particules de poudre ayant un diamètre < 75 µm représentent une proportion de 15 à 25 % du poids total des particules de poudre ;
des particules de poudre ayant un diamètre de 75 à 150 µm représentent une proportion de 30 à 50 % du poids total des particules de poudre ;
des particules de poudre ayant un diamètre de 150 à 250 µm représentent une proportion de 15 à 25 % du poids total des particules de poudre ;
des particules de poudre ayant un diamètre de 250 à 500 µm représentent une proportion de 15 à 25 % du poids total des particules de poudre ; et
des particules de poudre ayant un diamètre > 500 µm représentent une proportion < 2 % du poids total des particules de poudre.

# Fig. 1

# Fig. 2

# Fig. 3

# Fig. 3a

# Fig. 3b

# Fig. 4

a)

b)

Pille  15.0kV  5µm

# Fig. 5

(a)

(b)

(c)

(d)

# Fig. 6

$\sigma$ [N/mm$^2$]

$\sigma_u$

$\sigma_y$

40

41

E=d$\sigma$/d$\varepsilon$

$\varepsilon$ [%]

# Fig. 7

60

61

# Fig. 8

a)

30

b)

c)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 5451505 A
- US 6455157 B
- WO 0110464 A1
- US 4668523 A
- WO 2006047695 A

### In der Beschreibung aufgeführte Nicht-Patentliteratur

- **K. MARSHALL.** Tablet press fundaments. *Tablets & Capsules,* 2005, 6-11
- **N. A. ARMSTRONG.** Considerations of Compression Speed in Tablet Manufacture. *Pharmaceutical Technology,* September 1990, 106-114